Europäisches Patentamt

European Patent Office

Office européen des brevets

(11) Publication number: **0 455 923 A1**

## (12) EUROPEAN PATENT APPLICATION

(21) Application number: 90610034.2

(22) Date of filing: 09.05.90

(51) Int. Cl.⁵: **B02C 19/00**, B02C 23/10

(43) Date of publication of application:
**13.11.91 Bulletin 91/46**

(84) Designated Contracting States:
**DE DK ES FR GB IT NL SE**

(72) Inventor: **Aegidius Poul Erik,**
**Skovfogedvej 6**
**Anisse 3200 Helsinge(DK)**

(74) Representative: **Nielsen, Henrik Sten et al**
**Plougmann & Vingtoft Sankt Annae Plads 11**
**P.O. Box 3007**
**DK-1021 Copenhagen K(DK)**

(71) Applicant: **A/S N. Foss Electric**
**Slangerupgade 69**
**DK-3400 Hilleroed(DK)**

(54) An apparatus for grinding a sample of an organic material in a liquid and a system for analyzing a sample of an organic material.

(57) An apparatus (100) grinding a sample of an organic material in a liquid comprises a housing (104) which defines a cylindrical grinding chamber for receiving the sample and the liquid. The grinding chamber has a cylindrical inner wall and a bottom wall. The apparatus further comprises grinding means comprising a stationary grinding means (126) component and a rotatable grinding means component (120), which grinding means are arranged at the bottom wall of the grinding chamber, and motor means (106) rotating the rotatable grinding means component relative to the stationary grinding means component in a grinding operation. In the grinding operation, the organic material of the sample and the liquid is expelled in a liquid stream from the grinding means in a substantially vertical direction upwardly along the cylindrical inner wall of the grinding chamber, and the liquid stream is deflected by deflecting means (130) towards the centre of the cylindrical grinding chamber for recirculating the liquid and the organic material of the sample into engagement with the grinding means. A system for analyzing a sample of an organic material comprises, apart from the apparatus for grinding the sample (100), analyser means (300) for analyzing the content of the constituents of the liquid sample, and preferably further separator means (200) or separating the liquid sample from the extract or the emulsion.

Fig. 1

The present invention relates to the technical field of analyzing organic samples. More specifically, the present invention relates to a system for analyzing an organic material sample and an apparatus for grinding a sample of an organic material in a liquid such as a solvent, a decomposition agent or a combination thereof for generating an emulsion or an extract of the organic material in the liquid from which emulsion or extract a liquid sample may be withdrawn which liquid sample is to be analyzed.

It is a well-known technique within the art to analyze organic materials such as corn, grain, seed, oil seed, feed stuffs, meat samples, milk samples, etc., by grinding the sample in a liquid which constitutes a solvent or a decomposition agent for generating an emulsion or an extract of the sample, and by analyzing a liquid sample of the emulsion or extract. Since the emulsion or extract may contain solid particles it is often necessary to separate the solid particles from the emulsion or extract in order to eliminate the possibility or risk of blocking the analyzing apparatus by the introduction of the liquid sample therein. For separating the solid particles from the emulsion or extract, a centrifuge is often used.

Several grinding apparatuses are known, however, the known grinding apparatuses suffer from numerous disadvantages such as inefficient grinding operation and excessive noise generation. Since the grinding operation often generates unpleasant or even toxic gases it is highly desirable that the grinding apparatus may be sealed so that the unpleasant or even toxic gases are prevented from escaping to the atmosphere.

An example of a prior art grinding apparatus is disclosed in published German patent application DEOS 28 14 958, which discloses a grinding apparatus comprising a housing in which a rotatable impeller is journalled on a shaft of a motor. The impeller serves the purpose of causing particles contained in the liquid to be forced against a grinding surface, which may constitute a bottom surface of the housing or alternatively a peripheral inner wall part of the housing. In the grinding apparatus known from DEOS 28 14 958, the liquid containing the particles to be ground are simply thrown against the grinding surface which is to no extent sufficient for causing a complete grinding of particles of different hardnesses, such as on the one hand brittle or hard particles or products and on the other hand soft and complient particles or products. It is further to be realized that the apparatus known from DEOS 28 14 958 simply comprises an impeller which rotates within the liquid contained within the housing of the apparatus without causing any sufficient stirring or rearrangement of the liquid contained within the housing. Thus, the operation of the apparatus known from DEOS 28 14 958 is for most applications insufficient and uncapable of grinding soft and at the same time hard particles.

Grinder pumps are also known e.g. from US patent Nos. 3,726,486 and 4,108,386, which grinder pumps serve the purpose of grinding particles of a liquid stream which is pumped by means of the grinder pump. These grinder pumps serve the purpose of converting larger particles or substances contained within the liquid to be pumped by means of the grinder pump in question into minor particles or components which may be pumped by the grinder pump without causing clogging of any tubing system connected to the grinder pump in question. As will be readily understood, these grinder pumps are of a totally different structure as compared to the apparatus according to the present invention and further do not have to fulfill the extreme requirements as to their capability of grinding particles or materials of any hardness, size, composition, etc. into extremely small fractions which may be contained within a liquid introduced into the analyzing apparatus without causing blocking of the analyzing apparatus.

It should further be realized that the grinding apparatus of an analyzing system of the above type has to be capable of grinding brittle or hard products such as grain or seed products, and of grinding soft or complient products such as meat products which may even be sinewy and may give rise to problems such as blocking of the grinding apparatus or the like.

An object of the present invention is to provide an apparatus and a system of the above type, which apparatus and which system more efficiently than hitherto render possible the grinding and analyzing of samples of different products, such as meat products, milk products, grain, seed, corn, feed stuff products, or the like and which apparatus and which system further render it possible to carry out the grinding and analyzing operations in sealed environments in order to exclude unpleasant or even toxic or harmful gases from causing inconvenience or even harm to an operator or operators.

The above and other objects are obtained by means of an apparatus according to the present invention for grinding a sample of an organic material in a liquid and comprising:

a housing defining a substantially cylindrical grinding chamber for receiving said sample and said liquid, and being adapted to be used in a substantially upright position, said grinding chamber having a cylindrical inner wall and a bottom wall, said cylindrical inner wall and said bottom wall being substantially vertical and horizontal, respectively, when said housing is in said upright position,

grinding means comprising a stationary grinding means component and a rotatable grinding means component, said grinding means being arranged in said grinding chamber at said bottom wall thereof,

motor means for rotating said rotatable grinding means component relative to said stationary grinding means component in a grinding operation in which grinding operation said organic material of said sample and said liquid are forced by said rotatable grinding means component to move in a substantially radial direction at said bottom wall and into engagement with said grinding means, and in which grinding operation said organic material of said sample and said liquid is expelled in a liquid stream from said grinding means in a substantially vertical direction upwardly along said cylindrical inner wall of said grinding chamber, and

deflection means for deflecting said liquid stream from its substantially vertical direction towards the centre of said cylindrical grinding chamber.

The grinding means of the apparatus according to the present invention may be implemented in numerous ways, thus, the grinding means may comprise a toothed rim component. Alternatively, the grinding means of the apparatus according to the present invention may comprise e.g. a toothed disk, or the like.

In the presently preferred embodiment of the apparatus according to the present invention, the stationary grinding means component is a toothed rim component arranged at the cylindrical inner wall of the grinding chamber, and the rotatable grinding means component is a rotor means which is journalled centrally within the cylindrical grinding chamber and has an outer end part which defines a narrow annular space when the rotor means is rotating between the outer end part of the rotor means and the toothed rim component. Alternatively, the toothed rim component may be the rotatable grinding means component which cooperates with a stationary grinding means component. Alternatively, the stationary or rotatable grinding means component may be constituted by one of the above alternative components such as a toothed disk.

In accordance with a particular aspect of the present invention, the deflection means are constituted by a plurality of ribs arranged angularly spaced apart at the cylindrical inner wall of the cylindrical grinding chamber, which ribs define a pitch angle. The deflection means may, however, be implemented in numerous other ways, e.g. by deflection plates or the like.

In accordance with the above particular aspect of the present invention, the pitch angle defined by the ribs constituting the deflection means are adapted to the grinding means so as to cause the liquid stream which is generated by the grinding means as the liquid and organic material of the sample is expelled in a substantially vertical direction upwardly along the cylindrical inner wall of the grinding chamber to be deflected into direct engagement with the rotatable grinding means component which is preferably constituted by the above rotor means. A highly efficient grinding operation is consequently obtained since the liquid containing the sample material is efficiently recirculated into engagement with the rotatable grinding means component which causes the liquid and organic sample material to be forced into engagement with the grinding means for grinding therewith.

In accordance with a further aspect of the present invention, the apparatus according to the present invention is preferably provided with a lid for sealing the grinding chamber in relation to the environment so as to exclude gases such as unpleasant or even toxic or harmful gases from escaping from the grinding chamber.

The above object of the present invention is further obtained by means of a system according to the present invention for analyzing a sample of an organic material by grinding said sample in a liquid for generating an extract or an emulsion of constituents of said organic material in said liquid and by providing a liquid sample from said extract or emulsion and analyzing said liquid sample, said system comprising:

an apparatus for grinding said sample of said organic material in said liquid and comprising:

a housing defining a substantially cylindrical grinding chamber for receiving said sample and said liquid, and being adapted to be used in a substantially upright position, said grinding chamber having a cylindrical inner wall and a bottom wall, said cylindrical inner wall and said bottom wall being substantially vertical and horizontal, respectively, when said housing is in said upright position,

grinding means comprising a stationary grinding means component and a rotatable grinding means component, said grinding means being arranged in said grinding chamber at said bottom wall thereof,

motor means for rotating said rotatable grinding means component relative to said stationary grinding means component in a grinding operation in which grinding operation said organic material of said sample and said liquid are forced by said rotatable grinding means component to move in a substantially radial direction at said bottom wall and into engagement with said grinding means, and in which grinding operation said organic material of said sample and said liquid is expelled in a liquid stream from said grinding means in a substantially vertical direction upwardly along said cylindrical inner wall of said grinding chamber, and

deflection means for deflecting said liquid stream from its substantially vertical direction towards the centre of said cylindrical grinding chamber, and

an analyzer means for analyzing the content of said constituents of said liquid sample.

The system according to the present invention preferably further comprises a separator means for separating the liquid sample from the extract or emulsion, since in most applications the extract or emulsion comprises solid particles which have to be separated from the liquid sample to be introduced into the analyzing means. The separator means may be constituted by a filter means or preferably a centrifuge separator means, which renders it possible to provide a sealed system from which no gases may escape, and in which gas and liquid communication is established between the components of the system, i.e. between the grinding apparatus, the centrifuge separator, and the analyzer means by means of a sealed tubing system, and in accordance with the teaching of the present invention the analyzer means is preferably constituted by an IR analyzer means.

In accordance with the presently preferred embodiment of the system according to the invention, the centrifuge separator means comprises a centrifuge container body of a substantially disk-like configuration and having a bottom opening through which the extract or emulsion is introduced and through which the liquid sample is removed from the centrifuge container body.

In accordance with the presently preferred embodiment of the system, the grinding apparatus of the system is preferably further implemented in accordance with the above grinding apparatus according to the present invention. Numerous objects, features, and advantages of the present invention will be evident from the detailed description below.

Examples of analyzing organic material samples are:

The measurement of fat and protein in milk products or meat products by grinding the milk sample or meat sample in an aqueous solution of sodium hydroxide which aqueous solution may further comprise an emulsifier. The emulsion is transferred to a cuvette of an infrared spectrometer and the content of fat and protein is determined by determining the transmission and absorption at specific, characteristic wavelengths of the infrared wavelength spectrum.

The measurement of the content of fat or oil in corn, grain, seed, feed stuffs or the like by grinding a sample of the organic material in question in an extraction liquid, e.g. a hydrogen carbon composition, which extraction liquid according to health regulations must not be a toxic liquid or a liquid generating toxic gases, such as chlorinated hydrogen carbon compositions, by separating the solid particles from the liquid generated by the grinding of the sample in the extraction liquid by means of a centrifuge, a filter or another appropriate separator, by transferring a liquid sample from the liquid to a measuring cell, and by recording the content of fat or oil by measuring any characteristic indicative of the content of fat or oil in the sample, such as the density, the index of refraction, the absorption or transmission in a specific wavelength such as the infrared wavelength spectrum, etc.

The determination of the content of water of an organic sample such as a milk sample, a meat sample, a sample of corn, grain, seed, feed stuff, etc., by grinding the sample in question in an alcohol, by separating solid particles from the alcoholic liquid containing an extract of the sample, and by measuring the content of water in a liquid sample by means of an infrared analyzing apparatus.

The invention will now be further described with reference to the drawings, in which

Fig. 1 is a schematic view of an embodiment of a system according to the present invention and of grinding a solid organic material sample in a solvent for generating an emulsion of the organic material or generating an extract of the organic material, of separating constituents from the emulsion or extract of the organic material, and analyzing the constituents,

Fig. 2 a perspective, schematic and partly broken away view of an embodiment of a grinding apparatus constituting a component of the system shown in Fig. 1, and

Fig. 3 a diagrammatical view of two response curves of to separate samples which have been processed and analyzed in the system shown in Fig. 1.

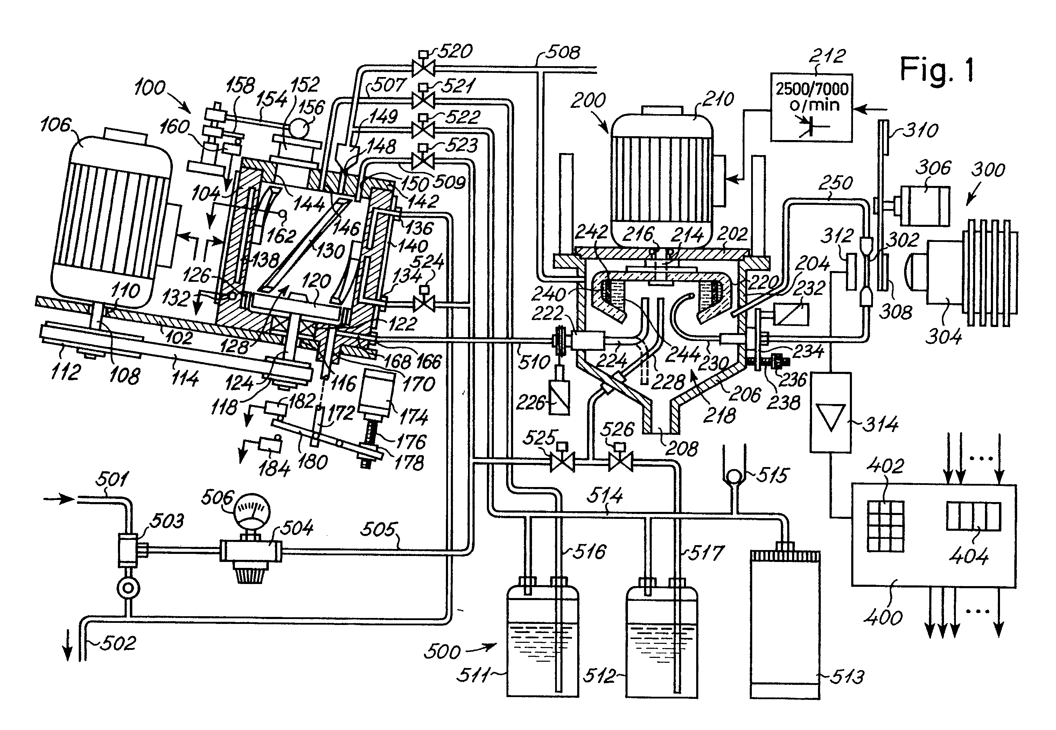

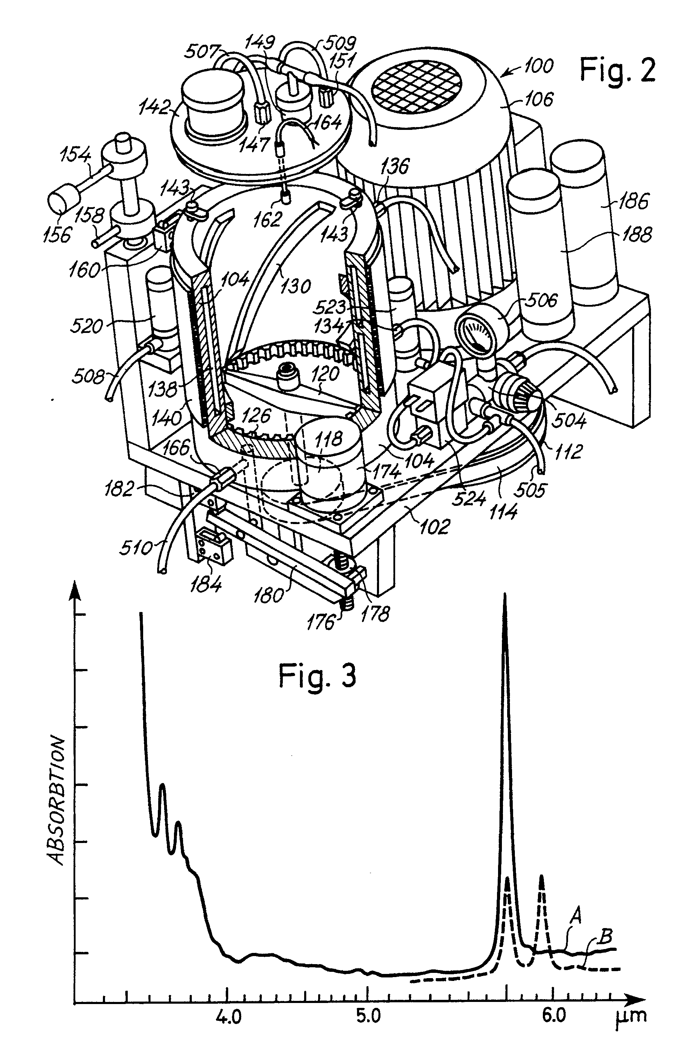

In Fig. 1 an embodiment of a system according to the present invention for carrying out the method according to the present invention is shown. Basically, the system is an automatized and sealed system from which no gases and no liquid may unintentionally escape and comprises a grinding apparatus 100 also shown in Fig. 2, a centrifuge separator 200, an infrared spectrometer 300, a central control and measuring computer 400 and a liquid and pressurized air assembly 500. The entire system is further connected to a water supply through a tube 501 and to a drain through a drain tube 502. The water supplied to the system through the water supply tube 501 is supplied to a pressure reduction valve 504 through a safety valve 503 which is further connected to the drain tube 502. The output of the pressure reduction valve 504 is connected to a tube 505 which constitutes an internal water supply tube of the entire system in which water is available at a fixed, predetermined pressure determined by the pressure reduction valve 504. The pressure setting of the pressure reduction valve is displayed on a pressure gauge 506.

The liquid container and pressurized air assembly 500 comprises a first liquid container 511 in which an antifoam agent is contained, a second liquid container 512 in which a saturated aqueous potassium carbonate solution is contained and a source 513 of pressurized air which is constituted by a pressurized air container connected to a compressor and which is connected to a pressurized air supply tube 514 to which a relief valve or safety valve 515 is connected so as to reduce the pressure of the pressurized air supplied from the source 513 to a predetermined upper or maximum pressure determined by the relief or safety valve 515. The pressurized air supply tube 514 constitutes an internal pressurized air supply tube of the system and is further connected to the first and second liquid containers 511 and 512, respectively, so as to maintain an elevated pressure in air spaces above the antifoam agent and the saturated aqueous potassium carbonate solution contained in the first liquid container 511 and in the second liquid container 512, respectively, by which elevated pressure the antifoam agent and the saturated aqueous potassium carbonate solution may be transferred through transfer tubes 516 and 517, respectively, which are immersed into the antifoam agent and the saturated aqueous potassium carbonate solution, respectively.

In the upper left hand part of Fig. 1, the grinding apparatus 100 is shown. The grinding apparatus 100 basically comprises a supporting frame 102 on which a cylindrical housing 104 is mounted. An AC motor 106 is further mounted on and supported by the supporting frame 102. An output shaft 108 of the AC motor 106 extends through an aperture 110 of the supporting frame 102 and is provided with a drive pulley 112 at the outermost or lower end of the output shaft 108 on which a drive pulley 112 and a drive belt 114 is mounted. Within the cylindrical housing 104, a rotor 120 is journalled in a journal bearing 122 constituted by a sealed roller bearing on a journal shaft 124 which extends through an aperture 116 of the supporting frame 102. At the outermost or lower end of the journal shaft 124, an idle pulley 118 is mounted on which the above drive belt 114 is also mounted. The dimensions of the drive pulley 112 and the idle pulley 118 and further the rotational speed of the AC motor 106 are adapted to each other so as to make the rotor 120 rotate at a speed of approximately 7000 rpm when the AC motor 106 is energized and rotates at its normal operational rotating speed.

At the lower end of the cylindrical housing 104, a toothed rim 126 is arranged which defines inner peripheral segments which are arranged juxtaposed the outer peripheral ends of the rotor 120 so that a small distance of approximately 0.03 mm is defined between the individual inner peripheral surface segments of the toothed rim 126 and the outer peripheral ends of the rotor 120. The toothed rim 126 is provided with a total of 36 notches which are mutually spaced apart an angular distance of 10°. Each of the notches has a circular cylindrical shape and defines a radial notch depth from the above peripheral inner surface segments of the toothed rim 126 of approximately 2 mm. The inner diameter defined by the above peripheral inner surface segments of the toothed rim 126 is approximately 77 mm. Although the shafts 108 and 124 are illustrated as being constituted by unsupported structures, at least the shaft 124 is preferably supported by a journal bearing at its outermost or lower end so that the shaft 124 is supported at opposite ends by two journal bearings one of which is the above journal bearing 122.

The shaft 124 is sealed relative to the cylindrical housing 104 by means of a gasket not shown on the drawings which is mounted on the shaft 124 above the journal bearing 122 and serves the purpose of preventing liquid from leaking from an inner space 128 defined within the cylindrical housing 104 to the journal bearing 122.

Apart from the toothed rim 126, the cylindrical housing 104 is at its inner peripheral surface provided with a total of four ribs or segments constituted by bodies of a plastics material which is resistant to the liquid to which the material is exposed, i.e. the consituents of the sample and the solvent or extraction liquid of the sample, e.g. alcohols and/or hydrocarbon compositions. The plastics material may e.g. be Delrin®. One of the ribs or segments is designated the reference numeral 130. The ribs 130 are arranged angularly spaced apart an angular distance of 90° and define a pitch angle of 45°.

The cylindrical housing 104 which is preferably made from stainless steel from which material the above rotor 120 and the above toothed rim 126 are also made, is provided with a water inlet 134 and a water outlet 136 which are connected to the above internal water supply tube 505 through a control valve 524 and the above drain tube 502, respectively. The water inlet 134 is connected to the water outlet 136 through an internal through-going conduit 138 of the cylindrical housing 104. The cylindrical housing 104 may consequently be cooled by the introduction of cooling water through the water inlet 134 from the water supply tube 505 through the control valve 524 and by conducting the cooling water through the internal through-going conduit 138 of the cylindrical body 104 to the water outlet 136 from which the cooling water is discharged to the drain tube 502. The temperature of the cylindrical housing 104 may be monitored by means of a temperature sensor such as a thermistor which is shown schematically in Fig. 1 and designated the reference numeral 162.

At its outer peripheral surface, the cylindrical housing 104 is provided with a heating element 140 which may be supplied with electrical power for heating the cylindrical housing 104 to a predetermined temperature which may be monitored by means of the temperature sensor 132 so that the cylindrical body 104 may be thermostated to a predetermined temperature by means of the heating element 140 and the temperature sensor 132 which are connected to the central computer 400.

An important aspect of the grinding apparatus 100 constituting an embodiment of the present invention is the fact that the grinding operation is performed in a sealed environment so that unpleasant or even harmful gases which may be produced in the grinding process are prevented from escaping from the sealed environment and are consequently prevented from causing any inconvenience or even harm to an operator or operators operating the grinding apparatus and the system shown in Fig. 1. For providing the sealed grinding environment, the cylindrical housing 104 is provided with a sealing lid 142 which seals the inner space 128 defined within the cylindrical housing 104. As is evident from Fig. 2, the sealing lid 143 is fixed relative to the cylindrical housing 104 by means of screws 142. In the sealing lid 142 an aperture 144 is provided together with three bores 146, 148 and 150. In the bore 146, an antifoam inlet tube 507 is mounted in a fitting which is shown in Fig. 2 and designated the reference numeral 147. The antifoam inlet tube 507 is connected to the above transfer tube 516 through a control valve 521. In the bore 148, a funnel-shaped expansion body 149 is arranged and is connected to the above pressurized air supply tube 514 through a control valve 522 and to a venting tube 508 through a control valve 520. In the bore 150, a tube 509 is arranged in a fitting shown in Fig. 2 and designated the reference numeral 151. The tube 509 is connected to the water supply tube 505 through a control valve 523. The tube 507, the body 149 and the tube 509 consequently serve the purpose of introducing the antifoam agent into the sealed inner space 128, of pressurizing the sealed inner space 128 or venting it, and of introducing rinsing water into the sealed inner space 128, respectively, as will be readily understood and further discussed below in connection with a detailed description of the operation of the grinding apparatus 100.

In the aperture 144 a plug body 152 is normally arranged, which by removal thereof allows for the introduction of a sample into the inner space 128. The plug body 152 is normally arranged in its sealing arrangement within the aperture 144 and is maintained in its sealing arrangement by means of a locking component 154 which is provided with an outer contact body 156. The component 154 is swingable from its normal position shown in Fig. 1 in which the contact body 156 bears against the upper surface of the plug body 152 and locks the plug body 152 in position in its sealing arrangement within the aperture 144, and a release position shown in Fig. 2 in which the plug body 152 may be removed from its sealing arrangement shown in Figs. 1 and 2 within the aperture 144 for providing access to the inner space 128. The position of the sealing component 154 is monitored by means of a microswitch 160 which generates a signal which is supplied to and detected by the central computer 400 when the sealing component 154 is in its normal locking position shown in Fig. 1. The microswitch 160 is activated by a protruding arm 158 of the swingable sealing component 154. The position of the sealing component 154 may alternatively be monitored by means of a reed switch which is activated by means of a magnet enclosed in the plug body 152. Thus, provided the plug body 152 is arranged in proximity to the reed switch, the reed switch generates a signal which indicates that the plug body 152 is in sealing arrangement in the aperture 144 and which is supplied to and detected by the central computer 400.

The temperature of the sample which is contained within the cylindrical housing 104 and is ground in a grinding operation performed by the grinding apparatus 100 as will be described in greater detail below, is monitored by means of a thermistor 162 which is shown schematically in Fig. 1 and in greater detail in Fig. 2. The thermistor 162 is supported in a through-going bore of the lid 142 and is provided with an electrical cable 164.

The material contained within the cylindrical housing 104 of the grinding apparatus 100 and/or any liquid, rinsing water, antifoam agent, etc., is expelled from the grinding apparatus 100 through a tube 510 which is connected to a liquid outlet fitting 166. The liquid outlet fitting 166 is connected to a radial bore 168 which is perpendicular to an axial bore 170. The axial bore 170 which communicates with the radial bore 168 provides liquid and air communication from the tube 510 to the inner space 128. The axial bore 170 is normally blocked by means of an axially movable stem 172 as shown in Fig. 1. The stem 172 is movable from its first, blocking position shown in Fig. 1 to a second, lowered position in which communication is established between the inner space 128 and the tube 510. The lowering of the stem 172 from its first position to its second position and further its raising from its second position to its first position is carried out by means of a DC motor which has an output spindle 176. The output spindle 176 meshes with a bushing which is mounted on a lever arm 180 which engages with the stem 172. The motions and the positions of the stem 172 are monitored by means of a first microswitch 182 and a second microswitch 184. In case the stem 172 is in its first position, the lever arm 180 is in contact with and activates the first microswitch 182. In case the stem is in the second position, the lever arm 180 is in contact with the second microswitch 184 which is consequently activated. The position of the stem 172 may consequently be monitored by the central control computer 400 and the motion of the stem from its first to its second position and vice versa may also be monitored by the central control computer 400 since none of the micro switches 182 and 184 generate control signals when the stem 172 is being moved from its first position to its second position or vice versa.

In Fig. 2, two capacitors 186 and 188 constitute starting capacitors of the AC motor 106 are further shown.

The grinding apparatus is operated in the following manner: A 20 g sample is introduced into the inner space 128 through the aperture 144 by the removal of the plug body 152. 120 ml solvent (organic or inorganic solvent, preferably a nontoxic, odourless hydrogen carbon composition having a low viscosity, a flash point of approximately 60°C, and a boiling point of approximately 180°C is also introduced into the inner space 128. Provided the sample is a meat sample, a decomposition agent is further introduced into the inner space 128, which decomposition agent may be constituted by 5 g NaOH. Before the introduction of the sample, the solvent and the decomposition agent, if any, into the inner space 128, the cylindrical housing 104 is preferably thermostated to a predetermined temperature such as a temperature of 45°C at which temperature the grinding and decomposition of the sample material is accelerated. During the introduction of the sample, the solvent and the decomposition agent, if any, the control valves 520, 521, 522 and 523 are closed.

The plug body 152 is rearranged in its sealing position shown in Figs. 1 and 2 and the sealing component 154 is arranged in its normal, locking position also shown in Fig. 1, in which locking position the microswitch 160 is activated. The motor 106 is energized for 2 minutes (for meat or milk products) or 3 minutes (for corn, grain, seed, feed stuff, etc.). During the grinding process, the control valves 520, 521, 522 and 523 are kept closed. By energizing the AC motor 106, the rotor 120 rotates as viewed from above in Fig. 2 in a counterclockwise direction. By its rotation in a counterclockwise direction, any liquid and solid material at the bottom of the cylindrical housing 104 is thrown radially outwards against the toothed rim 126 and any material in the centrifugal throw produced by the rotation of the rotor 120 is ground between the outer ends of the rotor 120 and the toothed rim 126. By the grinding process frictional heat is generated. Consequently, the apparatus is cooled by the introduction of cooling water into the throughgoing conduit 138 of the cylindrical housing 104. By the centrifugal reaction generated by the rotation of the rotor 120, the liquid and solid material is thrown upwards from the bottom part of the cylindrical housing 104 in a stream of liquid and solid material. The rotational speed of the rotor 120 and the pitch angle of the ribs or segments 130 are adapted to each other so that the stream of liquid and solid material is deflected inwardly towards the centre of the inner space 128. Due to the mutual adaptation of the pitch angle of the ribs or segments 130 and the rotational speed of the rotor 120, the deflection does not result in that the deflected liquid stream reaches the centre of the inner space 128 from all sides which inevitably would result in that liquid and solid material would simply be scattered within the inner space. The deflection of the stream of liquid and material is generated to such an extent that the liquid and the solid material drop down to the bottom of the cylindrical housing at a radial distance from the centre of the cylindrical housing 104. The material is consequently reimpacted by the rotor and thrown outwardly towards the toothed rim 126. The above reaction is further promoted by a suction effect generated by the rotor 120 as it throws the liquid and solid material outwardly towards the toothed rim 126. By the above deflection and suction effect, the liquid containing solid material is circulated in a basically closed loop circulation in which the liquid and the solid material is thrown outwardly impelled by the rotor 120, ground by the toothed rim 126, thrown upwardly and further deflected by the ribs 130 and sucked down towards the bottom of the inner space of the cylindrical housing 104.

By the adaptation of the pitch angle of the ribs 130 and the rotational speed of the rotor 120 to each other, an extremely efficient grinding operation is performed which is underlined by the fact that in the grinding process performed by the grinding apparatus 100 according to the present invention substantially no liquid and substantially no solid material is thrown so far upwardly that the liquid or the material is brought into contact with the lower side surface of the sealing lid 142. Thus, in the above described grinding process, all solid material is ground very efficiently.

After the performance of the above grinding process, the motor 106 is de-energized, and the DC motor 174 is energized for moving the stem 172 to its second, lowered position and for emptying the content from the inner space 128 of the grinding apparatus 100 through the tube 510. The emptying process is preferably performed while a minor excess pressure of approximately 0.15 bar is generated by opening the valve 522.

After the emptying of the content from the inner space 128 of the grinding apparatus 100, the apparatus is rinsed. Approximately 100 ml water is introduced into the inner space 128 through the tube 509 by opening the valve 523, closing the valve 522 and opening the valve 520. Thereupon, a small amount of antifoam agent, such as 500 $\mu$l, is introduced into the inner space 128 through the tube 507 by opening the valve 521. While introducing the antifoam agent, the valve 520 is optionally kept open, while the valves 522 and 523 are kept closed. Alternatively, the valve 520 may be closed while introducing the antifoam agent into the inner space 128. The AC motor 106 is then energized in its reverse rotational operational mode so that the rotor 120 is caused to rotate in its clockwise direction as viewed from above in Fig. 2. The rinsing liquid and the antifoam agent are consequently thrown up along the upper side surfaces of the ribs 130 and also flush the lower side surface of the sealing lid 142 for rinsing the inner side surfaces of the cylindrical housing 104 including the lower side surface of the sealing lid 142. Thereupon, the rinsing water and the antifoam agent is emptied through the tube 510 in the above described manner. The grinding apparatus 100 is now ready to perform another grinding operation. Alternatively, the grinding apparatus 100 may be rinsed once more in accordance with the above described rinsing procedure.

In the centre of Fig. 1, the centrifuge separator 200 is shown. The centrifuge separator 200 comprises a housing constituted by a top housing component 202 of a plane, circular configuration, a cylindrical housing component 204 which is rigidly connected at its upper rim to the top housing component 202 and a funnel-shaped housing component 206 which is integrally connected to the cylindrical housing component 204. At its central bottom part the funnel-shaped housing component 206 is provided with a waste outlet 208. An DC motor 210 is mounted on top of the top housing component 202. The DC motor 210 is controlled by a rotational speed controller 212 which is constituted by a thyristor controller which controls the DC motor 210 to rotate at a speed of 2500 rpm or 7000 rpm when energized. An output shaft 214 of the DC motor 210 extends through an aperture of the top housing component 202 into an inner space 218 defined within the housing composed of the housing components 202, 204 and 206. At an outer or lower end of the output shaft 214, a centrifuge container body 220 is mounted. The centrifuge container body 220 has a central, bottom opening through which access is obtained to an inner space defined within the centrifuge container body 220. The above described tube 510 through which liquid containing ground material and an emulsion and/or an extract is expelled from the grinding apparatus 100 extends into a swingable L-shaped tube 224. The L-shaped tube 224 is journalled in a bushing 222 which is arranged in an aperture of the cylindrical housing component 204. The L-shaped tube 224 is swingable from a position shown in solid line in Fig. 1 to a position shown in dotted line in Fig. 1 by means of a solenoid 226. An inlet tube 228 further extends into the inner space defined within the centrifuge container body 220 and is connectable to the water supply tube 505 through a control valve 525 or, alternatively, through a control 526 connectable to the transfer tube 517 emerged in the saturated aqueous potassium carbonate solution contained in the second liquid container 512. An U-shaped outlet tube 230 also extends into the inner space defined within the centrifuge container body 220 and is movable from a position shown in Fig. 1 to the right to a second position controlled by a solenoid motor 232 in which second position a plate 234 which is rigidly connected to the U-shaped outlet tube 230 bears against a nut 236. The nut 236 is mounted on a threaded stud 238.

The centrifuge separator 200 is operated in the following manner: Provided the centrifuge container body 220 is rinsed and clean, a small amount of saturated aqueous potassium carbonate solution is introduced into the inner space defined within the centrifuge container body 220 through the inlet tube 228 from the transfer tube 517 by opening the control valve 526. The saturated aqueous potassium carbonate solution which is introduced into the centrifuge container body 220 is thrown outwardly due to the centrifugal effect generated by the centrifuge container body 220 rotating at its high rotational speed of 7000 rpm driven by the DC motor 210. A saturated aqueous potassium carbonate solution film 240 is consequently arranged at the inner peripheral surface of the centrifuge container body 220. The saturated aqueous potassium carbonate solution film 240 constitutes a release film, as will be evident from the description below, and furthermore a film of a density of approximately 1.5 g/cm$^3$, i.e. a film of a higher density than most of the constituents and particles of the liquid which is introduced into the centrifuge container body 220. After the introduction of the saturated aqueous potassium carbonate solution into the inner space defined within the centrifuge container body 220 and the deposition of the saturated aqueous potassium carbonate solution film 240 within the centrifuge container body 220, the liquid expelled from the grinding apparatus 100 is introduced into the inner space defined within the centrifuge container 220 through the L-shaped tube 224 which is in its solid line position shown in Fig. 1. The liquid which is introduced into the inner space defined within the centrifuge container 220 contains the solvent, an emulsion of constituents of the organic sample material and/or an extract of the organic sample material, the decomposition agent and minor solid particles. In the infrared spectrometer 300 to be described below, the content of the emulsion and/or the extract contained in the liquid, is to be analyzed. However, the liquid to be analyzed in the infrared spectrometer 300 must not include solid particles which would inevitably block the infrared spectrometer and would furthermore influence the analyzing process due to light scattering effects. The solid particles are consequently to be separated from the liquid to be analyzed, which separation is carried out in the centrifuge separator 200.

The 7000 rpm rotation of the centrifuge container body 220 generates a virtual gravitational field of approximately 2000 G and consequently separates the liquid and any solid particles introduced into the centrifuge container body into high density and lower density layers. Since the solid particles have a higher density than the liquid, however a lower density than the saturated aqueous potassium carbonate film 240, the solid particles are separated from the liquid and arranged in a solid particle layer 242. Due to its lower density as compared to the solid particles the liquid is arranged in an innermost layer 244. Excess liquid is expelled through the lower central opening of the centrifuge container body 220 and further discharged through the waste opening 208. After rotating the centrifuge container body 220 at the high rotational speed of 7000 rpm for approximately 45 s., the DC motor 210 is slowly decelerated to its low rotational speed of 2500 rpm, whereupon the U-shaped outlet tube 230 is moved from its first, left hand position shown in Fig. 1 to its above-mentioned second, right hand position and consequently into contact with the liquid sample layer 244.

The liquid sample layer 244 is received in the tube 230, which has its outer end bent in a right angle as shown in Fig. 1 defining an outer end opening into which the liquid is pressed due to the velocity pressure which is generated as the liquid is rotated by the centrifuge container body 220. The liquid sample is transferred to a cuvette 302 which constitutes a measuring cuvette of the IR spectrometer 300 shown in the upper right hand part of Fig. 1. From the cuvette 302, a discharge tube 250 conducts the liquid from the cuvette 302 to the inner space 218 of the centrifuge separator 200 from which inner space the liquid is drained through the waste outlet 208.

After the transfer of the liquid sample layer 244 or at least part thereof to the cuvette 302 of the IR spectrometer 300, the centrifuge container body 220 is emptied by rapidly decelerating the centrifuge container body 220 to rest. By the rapid deceleration of the centrifuge container body 220, the material contained within the centrifuge container body 220, i.e. the saturated aqueous potassium carbonate solution film 240, the solid particle layer 242 and any remaining part of the liquid sample layer 244 are also decelerated. As mentioned above, the saturated aqueous potassium carbonate solution film 240 constitutes a release film which causes the solid particle layer 242 to slip relative to the centrifuge container body 220 which is decelerated rapidly, by which slipping process the solid particle layer 242 which is a very compact layer breaks up. Due the gravitational impact, the materials of the solid particle layer 242 and of the liquid sample layer 244 falls down through the bottom opening of the centrifuge container body 220 and are discharged through the waste outlet 208.

After the emptying of the centrifuge container body 220, the centrifuge container body 220 is preferably flushed with water which is introduced into the inner space defined within the centrifuge container body 220 through the control valve 525 which is opened and through the inlet tube 228. The flushing or rinsing of the centrifuge container body 220 is preferably carried out while the centrifuge container body 220 is rotating at its low rotational speed. After deceleration of the centrifuge container body 220 to rest, the centrifuge container body 220 is ready to receive another liquid charge from the grinding apparatus 100 for separating any solid particles from the liquid of the liquid charge prior to the introduction of the liquid into the IR spectrometer 300. Optionally, the above described flushing of the centrifuge container body 220 may be carried out more than once, e.g. two or three times.

Experiments have shown that for many applications, pure water may be used instead of the saturated aqueous potassium carbonate solution for generating a separation layer within the centrifuge container body 220. If pure water is used, the centrifuge container body 220 should be flushed at least two times, preferably three or more times. The pure water may be introduced into the centrifuge container body 220 through the valve 525 from the water supply tube 505 or, alternatively, the container 512 which in the above described embodiment comprises the saturated aqueous potassium carbonate solution may contain water to be introduced into the centrifuge container body 220 for generating the separation layer in the centrifuge separation operation carried out by means of the centrifuge separator 200.

In the upper right hand part of Fig. 1 the IR spectrometer 300 is shown. The IR spectrometer which is basically of a conventional type such as applicant's Milko-scan type, e.g. described in US patent No. 4,236,075 to which reference is made and which is hereby incorporated in the present specification by reference, centrally comprises the above mentioned cuvette 302. On the right hand side of the cuvette 302, an IR source 304 is arranged in front of which a shiftable IR filter assembly is arranged. The shiftable filter assembly comprises a motor 306 on the output shaft of which two IR filters are mounted.

In a first position of the motor 306, a first IR filter 308 is interposed between the IR source 304 and the cuvette 302, and in a second position of the motor 306, a second IR filter 310 is interposed between the IR source 304 and the cuvette 302. A third IR filter not shown in the drawings is furthermore provided, which third IR filter is interposed between the IR source 304 and the cuvette 302 in a third position of the motor 306. Provided the first IR filter 308, the second IR filter 310, or the third IR filter not shown in the drawings is arranged in the IR light path from the IR source 304 to the cuvette 302, IR radiation of a wavelength determined by the first IR filter 308, the second IR filter 310, or the third IR filter, respectively, is transmitted to the cuvette 302 and consequently to the liquid contained within the cuvette 302.

The IR transmission through the liquid contained in the cuvette 302 is detected by means of an IR detector 312 such as an IR resistor assembly, which is connected to a highly stable DC amplifier and A/D converter 314. An output of the DC amplifier and A/D converter 314 is connected to the central control computer 400. The first IR filter 308 defines a first wavelength and constitutes a reference IR filter, whereas the second and third filters define second and third IR wavelengths, respectively, which are characteristic to the absorption of two constituents which are to be determined. By comparison of the absorption at the first and second IR wavelengths and by comparison of the absorption at the first and third IR wavelengths the contents of the two constituents in question is determined by the computer 400.

As will be evident from the description above the computer 400 serves two main purposes, viz. the purpose of controlling the operation of the individual components of the system, such as the valves, motors, etc., of the grinding apparatus 100, the centrifuge separator 200, the IR spectrometer 300 and the liquid and pressurized air assembly 500. In accordance with a programme of the computer 400 and in response to control signals generated by detectors such as the above microswitches 160, 182 and 184 and detectors such as pressure and liquid detectors arranged in the tubes of the systems for generating signals indicative of the transfer of liquid through the tubes of the system, and the purpose of analyzing and comparing the measuring signals generated by the DC amplifier and A/D converter 314 in accordance with techniques well-known *per se*. The computer 400 is preferably constituted by a microprocessor which communicates with the above detectors, DC amplifier and A/D converter, etc. through appropriate interfaces also well-known in the art *per se*. As schematically shown in Fig. 1, the computer 400 is preferably contained in a housing in which a keyboard 402 is provided together with a display 404. The computer 400 preferably also communicates with a data logging apparatus or a printer as will be evident to a person having ordinary skill in the art.

In Fig. 3, a diagram is shown illustrating two curves, one in solid line and one in dotted line, designated the references A and B, respectively. The response curves A and B are obtained from two samples which have been processed in the grinding apparatus 100 of the system shown in Fig. 1 and in the centrifuge separator 200 of the system shown in Fig. 1 and are recorded by means of an IR spectrometer. The curves A and B represent the absorption of the corresponding samples versus wavelength. The ordinate axis of the diagram shown in Fig. 3 represents the absorption A. Below a wavelength of 4.0 $\mu$m, the response curve A exhibits a high absorption response which is characteristic to the organic solvent of the liquid. The response curve A exhibits a spike at a wavelength of 5.7 $\mu$m, which is characteristic to a constituent of the sample in question, viz. a constituent comprising a carbonyl group. The response curve B is characteristic to a sample of rancid fats such as rancid butter or tainted fish.

In the IR spectrometer 300, the above described second and third IR filters preferably have wavelengths corresponding to the spikes of the response curve B, i.e. wavelengths of approximately 5.7 $\mu$m and 5.9 $\mu$m, respectively, whereas the above described first IR filter preferably has a wavelength below 5.7 $\mu$m, such as a wavelength of approximately 5.6 $\mu$m.

Although the present invention has been described above with reference to the drawings in which specific detailed embodiments of the apparatus and system according to the present invention are disclosed, the above description and the technical discussion are not to be construed limiting the present invention in any way.

**Claims**

1. An apparatus for grinding a sample of an organic material in a liquid and comprising:

a housing defining a substantially cylindrical grinding chamber for receiving said sample and said liquid, and being adapted to be used in a substantially upright position, said grinding chamber having a cylindrical inner wall and a bottom wall, said cylindrical inner wall and said bottom wall being substantially vertical and horizontal, respectively, when said housing is in said upright position,

grinding means comprising a stationary grinding means component and a rotatable grinding means component, said grinding means being arranged in said grinding chamber at said bottom wall thereof,

motor means for rotating said rotatable grinding means component relative to said stationary grinding means component in a grinding operation in which grinding operation said organic material of said sample and said liquid are forced by said rotatable grinding means component to move in a substantially radial direction at said bottom wall and into engagement with said grinding means, and in which grinding operation said organic material of said sample and said liquid is expelled in a liquid stream from said grinding means in a substantially vertical direction upwardly along said cylindrical inner wall of said grinding chamber, and

deflection means for deflecting said liquid stream from its substantially vertical direction towards the centre of said cylindrical grinding chamber.

2. An apparatus according to claim 1, said grinding means comprising a toothed rim component.

3. An apparatus according to claim 1, said stationary grinding means component being a toothed rim component arranged at said cylindrical inner wall of said grinding chamber, and said rotatable grinding means component being a rotor means journalled centrally within said cylindrical grinding chamber and having an outer end part which defines a narrow annular space when said rotor means is rotating between said outer end part and said toothed rim component.

4. An apparatus according to any of the claims 1-3, said deflection means being constituted by a plurality of ribs arranged angularly spaced apart at said cylindrical inner wall and defining a pitch angle.

5. An apparatus according to claim 4, said pitch angle being adapted to said grinding means for causing said liquid stream to be deflected into direct engagement with said rotatable grinding means component.

6. An apparatus according to any of the claims 1-5, further comprising a lid for sealing said grinding chamber in relation to the environment.

7. A system for analyzing a sample of an organic material by grinding said sample in a liquid for generating an extract or an emulsion of constituents of said organic material in said liquid and by providing a liquid sample from said extract or emulsion and analyzing said liquid sample, said system comprising:

an apparatus for grinding said sample of said organic material in said liquid and comprising:

a housing defining a substantially cylindrical grinding chamber for receiving said sample and said liquid, and being adapted to be used in a substantially upright position, said grinding chamber having a cylindrical inner wall and a bottom wall, said cylindrical inner wall and said bottom wall being substantially vertical and horizontal, respectively, when said housing is in said upright position,

grinding means comprising a stationary grinding means component and a rotatable grinding means component, said grinding means being arranged in said grinding chamber at said bottom wall thereof,

motor means for rotating said rotatable grinding means component relative to said stationary grinding means component in a grinding operation in which grinding operation said organic material of said sample and said liquid are forced by said rotatable grinding means component to move in a substantially radial direction at said bottom wall and into engagement with said grinding means, and in which grinding operation said organic material of said sample and said liquid is expelled in a liquid stream from said grinding means in a substantially vertical direction upwardly along said cylindrical inner wall of said grinding chamber, and

deflection means for deflecting said liquid stream from its substantially vertical direction towards the centre of said cylindrical grinding chamber, and

an analyzer means for analyzing the content of said constituents of said liquid sample.

8. A system according to claim 7, further comprising separator means for separating said liquid sample from said extract or said emulsion.

9. A system according to claim 7 or 8, said separator means being constituted by centrifuge separator means, and said analyzer means being constituted by an IR analyzer means.

10. A system according to claim 9, said centrifuge separator means comprising a centrifuge container body of a substantially disk-like configuration and having a bottom opening through which said extract or emulsion is introduced and through which said liquid sample is removed from said centrifuge container body.

11. A system according to any of the claims 7-10, said grinding apparatus having any of the characteristics of the apparatus according to any of the claims 2-6.

Fig. 1

Fig. 2

Fig. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-4015781 (BECK)<br>* column 3, line 36 - column 4, line 33; figures 1, 2, 5, 6 * | 1-6 | B02C19/00<br>B02C23/10 |
| A | | 7, 11 | |
| A | US-A-4767069 (CHONG S. KIM)<br>* the whole document * | 1-7, 11 | |
| A | US-A-4133487 (JUAN E. LANIER)<br>* abstract; figure 1 * | 1-3, 6 | |
| A | EP-A-356649 (ORTMAYER, RUDOLF-MAXIMILIAN)<br>* the whole document * | 1, 7-11 | |
| A,D | US-A-4236075 (NEXO ET AL.)<br>* abstract; figures 1, 2 * | 7 | |
| A,D | DE-A-2814958 (HOORN) | | |

TECHNICAL FIELDS
SEARCHED (Int. Cl.5 )

B02C
B04B

The present search report has been drawn up for all claims

| Place of search | Date of completion of the search | Examiner |
|---|---|---|
| BERLIN | 14 DECEMBER 1990 | CUNY, J |

EPO FORM 1503 03.82 (P0401)